(19) Europäisches Patentamt
European Patent Office
Office européen des brevets

(11) EP 3 317 247 B1

(12) **FASCICULE DE BREVET EUROPEEN**

(45) Date de publication et mention de la délivrance du brevet:
**08.05.2019 Bulletin 2019/19**

(21) Numéro de dépôt: **16732992.9**

(22) Date de dépôt: **15.06.2016**

(51) Int Cl.:
*C07C 67/347* (2006.01)

(86) Numéro de dépôt international:
**PCT/EP2016/063718**

(87) Numéro de publication internationale:
**WO 2017/001194 (05.01.2017 Gazette 2017/01)**

(54) **PROCÉDÉ DE PRÉPARATION DE POLYOLS**

VERFAHREN ZUR HERSTELLUNG VON POLYOLEN

PROCESS FOR PREPARING POLYOLS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.06.2015 FR 1501373**

(43) Date de publication de la demande:
**09.05.2018 Bulletin 2018/19**

(73) Titulaires:
- **Pivert S.A.S.**
  **60280 Venette (FR)**
- **Centre National de la Recherche Scientifique CNRS**
  **75794 Paris Cedex 16 (FR)**
- **Université d'Artois**
  **62030 Arras Cedex (FR)**

(72) Inventeurs:
- **HAPIOT, Frédéric**
  **59710 Ennevelin (FR)**
- **MONFLIER, Eric**
  **59110 La Madeleine (FR)**
- **VANBESIEN, Théodore**
  **CS 50149 Venette**
  **60201 Compiègne Cedex (FR)**

(74) Mandataire: **Connor, Marco Tom et al**
**Pecher & Partners**
**Rue Louis de Geer, 6**
**1348 Louvain-la-Neuve (BE)**

(56) Documents cités:
**WO-A1-03/093215** **GB-A- 2 046 266**
**US-A1- 2005 070 620**

- **LAWRENCE L. W. CHEUNG ET AL: "Synthesis of Alcohols via a Rhodium-Catalyzed Hydroformylation- Reduction Sequence using Tertiary Bidentate Amine Ligands", ADVANCED SYNTHESIS & CATALYSIS, vol. 354, no. 10, 9 juillet 2012 (2012-07-09), pages 2019-2022, XP055265931, DE ISSN: 1615-4150, DOI: 10.1002/adsc.201200053**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

### Domaine de l'invention

L'invention concerne un procédé de préparation de polyols, de préférence à partir de triglycérides. En particulier, l'invention se rapporte à un procédé de préparation de polyols en une seule étape par réaction d'hydrohydroxyméthylation ou d'hydroformylation réductrice.

### Arrière-plan technologique de l'invention

Les polyols sont généralement produits à partir du pétrole. Les polyols sont utilisés dans de nombreux domaines d'applications tels que les textiles, les plastiques, la chimie, l'industrie manufacturière ou l'industrie cosmétique. Les polyols sont notamment utilisés dans la préparation de revêtements, d'adhésifs, d'élastomères, de résines ou de mousses.

Les polyols sont généralement préparés via une réaction d'hydroformylation d'alcènes. Toutefois, la majeure partie des procédés décrits dans l'art antérieur sur ce type d'approche utilisent des ligands difficiles à manipuler à l'air tels que des trialkylphosphines ou des phosphites qui se dégradent dans l'eau. Une alternative plus simple consiste à utiliser des amines comme ligands. L'utilisation d'amines tertiaires a été décrite dans la littérature pour convertir des alcènes terminaux en alcools par l'intermédiaire d'une réaction d'hydroformylation réductrice (Morales Torres et al., Catal. Sci. Technol., 2015, 5, 34-54). Diverses études systématiques ont montré l'influence d'amine, en termes de structure, de basicité et de quantité, sur la réaction d'hydroformylation au rhodium (Hunter et al., Appl. Catal., 1985, 19, 275-285). L'utilisation la plus récente de ce type de système catalytique remonte à 2012 par Alper et al., (Adv. Synth. Catal., 2012, 354, 2019-2022) sur la synthèse d'alcools terminaux à partir du styrène en présence de diamines tertiaires comme ligand. La production d'alcool par ce procédé utilisant les systèmes catalytiques rhodium-amine a également été décrite dans EP 0014225 et US 4,197,414 mais uniquement à partir d'oléfines légères de type 1-hexène.

Pour des oléfines plus lourdes, la synthèse de polyols s'effectue préférentiellement par une réaction d'époxydation comme décrit par WO2006/0112344.

Par ailleurs, WO03093215 divulgue la synthèse de polyols à partir d'un acide gras insaturé en présence d'un catalyseur rhodium-phosphine.

### Résumé de l'invention

La présente invention permet la préparation de polyols à partir de composés biosourcés, par exemple à partir d'une huile végétale. Le procédé est particulièrement intéressant pour la préparation sélective de polyols à partir de triglycérides avec un rendement et une sélectivité élevés.

La présente invention concerne un procédé de préparation de polyols à partir d'une composition A comprenant un ou plusieurs composés de formule (I)

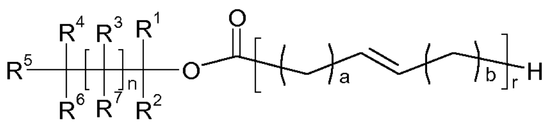

(I)

dans laquelle

$R^1$, $R^2$, $R^3$, $R^4$, $R^5$, $R^6$ et $R^7$ sont indépendamment les uns des autres, et pour $R^3$ et $R^7$ indépendamment pour chacune des unités n, choisis parmi le groupe consistant en H, $-OR^{15}$, alkyle en $C_1$-$C_{10}$ substitué ou non par un ou plusieurs groupes $-OR^{15}$, aryle en $C_6$-$C_{12}$ substitué ou non par un ou plusieurs groupes - $OR^{15}$, ou cycloalkyle en $C_3$-$C_{10}$ substitué ou non par un ou plusieurs groupes - $OR^{15}$, ou un groupement de formule (Ia)

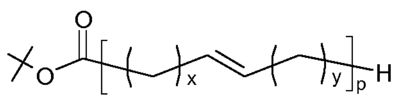

(Ia)

$R^{15}$ représente H ou alkyle en $C_1$-$C_{10}$ substitué ou non par un ou plusieurs groupes -OH

a, b, x et y sont indépendamment les uns des autres, indépendamment pour chaque groupement de formule (Ia), indépendamment pour chaque unité $[(CH_2)_a$-$C=C$-$(CH_2)_b]_r$ et indépendamment pour chaque unité $[(CH_2)_x$-$C=C$-$(CH_2)_y]_p$, un nombre entier compris entre 0 et 20, avantageusement entre 0 et 15, de préférence entre 0 et 12 ;

r est un nombre entier compris entre 1 et 10, avantageusement entre 1 et 5 ;

p est un nombre entier compris entre 1 et 10, avantageusement entre 1 et 5 ;

n est un nombre entier entre 1 et 7 ;

ledit procédé comprenant une étape a) de mise en présence sous agitation et sous une atmosphère d'hydrogène et de monoxyde de carbone :

- d'au moins un précatalyseur étant un complexe comprenant un métal de transition choisi parmi la colonne 9 du tableau périodique des éléments,
- d'une amine tertiaire, ou un sel d'ammonium non quaternaire de celle-ci, de formule $NR^8R^9R^{10}$ dans laquelle $R^8$, $R^9$ et $R^{10}$ représentent indépendamment les uns des autres un alkyle en $C_1$-$C_{10}$, aryle en $C_6$-$C_{12}$, cycloalkyle en $C_3$-$C_{10}$ ou $R^8$ et $R^9$ forment ensemble avec l'atome d'azote auquel ils sont attachés un hétérocycle à quatre, cinq ou six chaînons, et
- de ladite composition A comprenant un ou plusieurs composés de formule (I).

Le présent procédé permet la formation d'un ou plusieurs composés issus desdits un ou plusieurs composés de formule (I) dans lequel, pour tout ou partie des doubles liaisons carbone-carbone, un atome de carbone des doubles liaisons carbone-carbone du composé de formule (I) a été substitué par un groupement $-CH_2OH$, l'autre atome de carbone de cette même double liaison carbone étant substitué par un hydrogène.

## Brève description des figures

La fig. 1 représente un schéma de la synthèse de polyols selon un mode de réalisation particulier de l'invention.

La fig. 2 représente un spectre $^1H$ RMN de la trioléine.

La fig. 3 représente un spectre 1H RMN du produit obtenu par le procédé selon un mode de réalisation particulier à partir de la trioléine.

## Description détaillée de la présente invention

Le terme « substitué » tel qu'utilisé dans la présente invention signifie que un ou plusieurs atomes d'hydrogène du groupement auquel le terme « substitué » fait référence est remplacé par un des substituants nommés à condition que la valence normale de l'atome sur lequel la substitution est considérée n'est pas dépassée et que la substitution résulte en un composé chimique stable, c'est-à-dire un composé suffisamment robuste pour être isolé d'un mélange réactionnel.

Le terme « alkyl » se réfère à des chaînes hydrocarbonées linéaires ou branchées contenant le nombre spécifié d'atomes de carbone. Par exemple, alkyle en $C_1$-$C_6$ signifie un groupe alkyle linéaire ou branché contenant au moins 1, et au plus, 6 atomes de carbone. Le groupement alkyle peut être substitué par un groupement aryle non substitué, halogène, $NO_2$, CN, $SO_3H$, OH, alkoxy en $C_1$-$C_{10}$, un groupement carbonyle ou carboxyle. Le terme « aryle » se réfère à un cycle hydrocarboné aromatique contenant le nombre spécifié d'atomes de carbone substitué ou non par un alkyle $C_1$-$C_{10}$ non substitué, halogène, $NO_2$, CN, $SO_3H$, un groupement carbonyle, carboxyle, OH, alkoxy en $C_1$-$C_{10}$. Par exemple, aryle peut être un phényle, naphtyle, anthracyle ou phénanthryle. Le terme « cycloalkyle » se réfère à un cycle hydrocarboné non aromatique monocyclique ou polycyclique condensé comportant le nombre spécifié d'atomes de carbone. Par exemple, cycloalkyle comprend le cyclopropyle, cyclobutyle, cyclopentyle, cyclohexyle, cycloheptyle ou cyclooctyle. Le terme « hétérocycle » se réfère à un cycle hydrocarboné non aromatique monocyclique ou polycyclique condensé comportant le nombre de chaînons spécifié, dans lequel au moins un des atomes de carbone est remplacé par un atome de phosphore, de soufre, d'azote ou d'oxygène. En particulier, le terme hétérocycle tel qu'utilisé dans la présente invention se réfère à un cycle hydrocarboné non aromatique monocyclique ou polycyclique condensé comportant le nombre de chaînons spécifié et dans lequel au moins un des atomes de carbone est remplacé par un atome d'azote.

Selon la présente invention, un procédé de préparation de polyols est fourni. Le présent procédé de préparation de polyols est effectué à partir d'une composition A comprenant un ou plusieurs composés de formule (I)

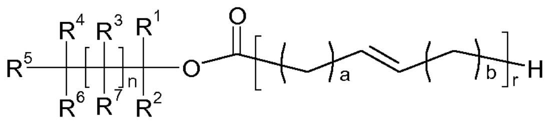

(I)

dans laquelle

$R^1$, $R^2$, $R^3$, $R^4$, $R^5$, $R^6$ et $R^7$ sont indépendamment les uns des autres, et pour $R^3$ et $R^7$ indépendamment pour chacune des unités $[CR^3R^7]_n$, choisis parmi le groupe consistant en H, $-OR^{15}$, alkyle en $C_1$-$C_{10}$ substitué ou non par un ou plusieurs groupes $-OR^{15}$, aryle en $C_6$-$C_{12}$ substitué ou non par un ou plusieurs groupes $-OR^{15}$, ou cycloalkyle en $C_3$-$C_{10}$ substitué ou non par un ou plusieurs groupes $-OR^{15}$, ou un groupement de formule (Ia)

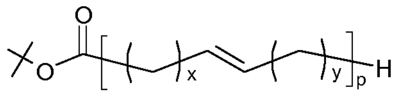

(Ia)

$R^{15}$ représente H ou alkyle en $C_1$-$C_{10}$ substitué ou non par un ou plusieurs groupes -OH ;
a, b, x et y sont indépendamment les uns des autres, indépendamment pour chaque groupement de formule (Ia), indépendamment pour chaque unité $[(CH_2)_a\text{-}C\text{=}C\text{-}(CH_2)_b]_r$ et indépendamment pour chaque unité $[(CH_2)_x\text{-}C\text{=}C\text{-}(CH_2)_y]_p$, un nombre entier compris entre 0 et 20, avantageusement entre 0 et 15, de préférence entre 0 et 12 ;
r est un nombre entier compris entre 1 et 10, avantageusement entre 1 et 5 ;
p est un nombre entier compris entre 1 et 10, avantageusement entre 1 et 5 ;
n est un nombre entier entre 1 et 7 ;
ledit procédé comprenant une étape a) de mise en présence sous agitation et sous une atmosphère d'hydrogène et de monoxyde de carbone :

- d'au moins un précatalyseur étant un complexe comprenant un métal de transition choisi parmi la colonne 9 du tableau périodique des éléments,
- d'une amine tertiaire, ou un sel d'ammonium non quaternaire de celle-ci, de formule $NR^8R^9R^{10}$ dans laquelle $R^8$, $R^9$ et $R^{10}$ représentent indépendamment les uns des autres un alkyle en $C_1$-$C_{10}$, aryle en $C_6$-$C_{12}$, cycloalkyle en $C_3$-$C_{10}$ ou $R^8$ et $R^9$ forment ensemble avec l'atome d'azote auquel ils sont attachés un hétérocycle à quatre, cinq ou six chaînons,
- de ladite composition A comprenant un ou plusieurs composés de formule (I).

Les doubles liaisons carbone-carbone contenues dans le(s) composé(s) de formule (I), (Ia) ou (II) telles que décrites dans la présente invention peuvent être de configuration cis ou trans. Le terme double(s) liaison(s) carbone-carbone englobe les deux configurations.

La figure 1 illustre le procédé selon un mode de réalisation particulier de la présente invention faisant intervenir une réaction d'hydrohydroxyméthylation. Le composé (I) est représenté par la trioléine comprenant trois doubles liaisons carbone-carbone. Le procédé selon la présente invention permet ainsi la préparation d'un polyol dans lequel les trois doubles liaisons carbone-carbone sont hydrohydroxyméthylées. Le groupement $-CH_2OH$ peut être porté pour chacune des doubles liaisons carbone-carbone par l'un quelconque des atomes de carbone C1 ou C2, C'1 ou C'2, C"1 ou C"2.

Selon un mode de réalisation préféré, ladite composition A peut comprendre au moins 20% en poids desdits un ou plusieurs composés de formule (I) sur base du poids total de la composition A, avantageusement au moins 30% en poids, de préférence au moins 40% en poids, plus préférentiellement au moins 50% en poids, en particulier au moins 60% en poids, plus particulièrement au moins 70% en poids, de manière privilégiée au moins 80% en poids.

De préférence, a et x peuvent être indépendamment l'un de l'autre, indépendamment pour chaque groupement de formule (Ia), indépendamment pour chaque unité $[(CH_2)_a\text{-}C\text{=}C\text{-}(CH_2)_b]_r$ et indépendamment pour chaque unité $[(CH_2)_x\text{-}C\text{=}C\text{-}(CH_2)_y]_p$ un nombre entier compris entre 1 et 12, avantageusement entre 2 et 10, de préférence entre 3 et 9, en particulier entre 3 et 8 et plus particulièrement entre 4 et 8.

De préférence, b et y peuvent être indépendamment l'un de l'autre, indépendamment pour chaque groupement de formule (Ia), indépendamment pour chaque unité $[(CH_2)_a\text{-}C\text{=}C\text{-}(CH_2)_b]_r$ et indépendamment pour chaque unité $[(CH_2)_x\text{-}C\text{=}C\text{-}(CH_2)_y]_p$ un nombre entier compris entre 0 et 12, avantageusement entre 0 et 10, de préférence entre 0

et 9, en particulier entre 2 et 9, et plus particulièrement entre 3 et 8.

De préférence, n peut être un nombre entier compris entre 1 et 7, avantageusement entre 1 et 5, de préférence entre 1 et 4, en particulier entre 1 et 3, et plus particulièrement n est 1.

De préférence, p peut être un nombre entier compris entre 1 et 5, avantageusement entre 1 et 4, de préférence entre 1 et 3. En particulier, p est 1, 2 ou 3, plus particulièrement, p est 1 ou 2.

De préférence, r peut être un nombre entier compris entre 1 et 5, avantageusement entre 1 et 4, de préférence entre 1 et 3. En particulier, r est 1, 2 ou 3, plus particulièrement, r est 1 ou 2.

Avantageusement, dans lesdits un ou plusieurs composés de formule (I), au moins un des substituants $R^1$, $R^2$, $R^3$, $R^4$, $R^5$, $R^6$ et $R^7$ est un groupement de formule (Ia). De préférence, dans lesdits un ou plusieurs composés de formule (I), au moins deux des substituants $R^1$, $R^2$, $R^3$, $R^4$, $R^5$, $R^6$ et $R^7$ est un groupement de formule (Ia).

Selon un mode de réalisation préféré, lesdits un ou plusieurs composés de formule (I) sont de formule (II)

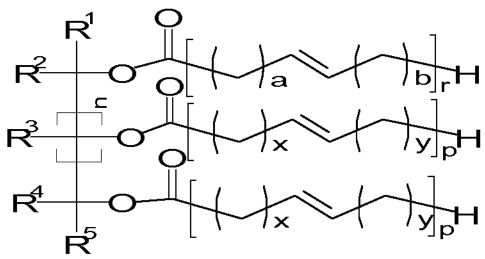

(II)

,

dans laquelle

a, b, x et y sont indépendamment les uns des autres, indépendamment pour chaque groupement de formule (Ia), indépendamment pour chaque unité $[(CH_2)_a\text{-}C{=}C\text{-}(CH_2)_b]_r$ et indépendamment pour chaque unité $[(CH_2)_x\text{-}C{=}C\text{-}(CH_2)_y]_p$, un nombre entier compris entre 0 et 20, avantageusement entre 0 et 15, de préférence entre 0 et 12 ;

r est un nombre entier compris entre 1 et 10, avantageusement entre 1 et 5 ;

p est un nombre entier compris entre 1 et 10, avantageusement entre 1 et 5 ;

n est un nombre entier entre 1 et 7, de préférence entre 1 et 4 ;

$R^1$, $R^2$, $R^3$, $R^4$ et $R^5$ sont indépendamment les uns des autres, et pour $R^3$ indépendamment pour chacune des unités n, choisis parmi le groupe consistant en H, $-OR^{15}$, un groupement de formule (Ia) tel que décrit ci-dessus, alkyle en $C_1$-$C_{10}$ substitué ou non par un ou plusieurs groupes $-OR^{15}$, aryle en $C_6$-$C_{12}$ substitué ou non par un ou plusieurs groupes $-OR^{15}$, ou cycloalkyle en $C_3$-$C_{10}$ substitué ou non par un ou plusieurs groupes $-OR^{15}$, dans lequel $R^{15}$ représente H ou alkyle en $C_1$-$C_{10}$ substitué ou non par un ou plusieurs groupes -OH.

Dans lesdits un ou plusieurs composés de formule (II), a et x peuvent être indépendamment l'un de l'autre, indépendamment pour chaque unité $[(CH_2)_x\text{-}C{=}C\text{-}(CH_2)_y]_p$ et indépendamment pour chaque unité $[(CH_2)_a\text{-}C{=}C\text{-}(CH_2)_b]_r$, un nombre entier compris entre 1 et 12, avantageusement entre 2 et 10, de préférence entre 3 et 9, en particulier entre 3 et 8 et plus particulièrement entre 4 et 8.

Dans lesdits un ou plusieurs composés de formule (II), b et y peuvent être indépendamment l'un de l'autre, indépendamment pour chaque unité $[(CH_2)_x\text{-}C{=}C\text{-}(CH_2)_y]_p$ et indépendamment pour chaque unité $[(CH_2)_a\text{-}C{=}C\text{-}(CH_2)_b]_r$, un nombre entier compris entre 0 et 12, avantageusement entre 0 et 10, de préférence entre 0 et 9, en particulier entre 2 et 9, et plus particulièrement entre 3 et 8.

Dans lesdits un ou plusieurs composés de formule (II), n peut être un nombre entier compris entre 1 et 7, avantageusement entre 1 et 5, de préférence entre 1 et 4, en particulier entre 1 et 3, et plus particulièrement n est 1.

Dans lesdits un ou plusieurs composés de formule (II), p peut être un nombre entier compris entre 1 et 5, avantageusement entre 1 et 4, de préférence entre 1 et 3. En particulier p est 1, 2 ou 3, plus particulièrement, p est 1 ou 2.

Dans lesdits un ou plusieurs composés de formule (II), r peut être un nombre entier compris entre 1 et 5, avantageusement entre 1 et 4, de préférence entre 1 et 3. En particulier r est 1, 2 ou 3, plus particulièrement, r est 1 ou 2.

De préférence, dans lesdits un ou plusieurs composés de formule (II), les substituants $R^1$, $R^2$, $R^3$, $R^4$ et $R^5$ peuvent être indépendamment les uns des autres, et pour $R^3$ indépendamment pour chacune des unités n, choisis parmi le groupe consistant en H, $-OR^{15}$, un groupement de formule (Ia) tel que décrit ci-dessus, alkyle en $C_1$-$C_{10}$ substitué ou non par un ou plusieurs groupes $-OR^{15}$, dans lequel $R^{15}$ représente H ou alkyle en $C_1$-$C_{10}$ substitué ou non par un ou plusieurs groupes -OH.

En particulier, dans lesdits un ou plusieurs composés de formule (II), les substituants $R^1$, $R^2$, $R^3$, $R^4$ et $R^5$ peuvent être indépendamment les uns des autres, et pour $R^3$ indépendamment pour chacune des unités n, choisis parmi le groupe consistant en H, alkyle en $C_1$-$C_{10}$ substitué ou non par un ou plusieurs groupes $-OR^{15}$, dans lequel $R^{15}$ représente H.

Plus particulièrement, dans lesdits un ou plusieurs composés de formule (II), les substituants $R^1$, $R^2$, $R^3$, $R^4$ et $R^5$ sont un hydrogène.

Ainsi, selon un mode de réalisation préféré, lesdits un ou plusieurs composés sont de formule (II) dans laquelle

a et x peuvent être indépendamment l'un de l'autre, indépendamment pour chaque unité $[(CH_2)_x\text{-}C{=}C\text{-}(CH_2)_y]_p$ et indépendamment pour chaque unité $[(CH_2)_a\text{-}C{=}C\text{-}(CH_2)_b]_r$, un nombre entier compris entre 1 et 12, avantageusement entre 2 et 10, de préférence entre 3 et 9, en particulier entre 3 et 8 et plus particulièrement entre 4 et 8 ;
b et y peuvent être indépendamment l'un de l'autre, indépendamment pour chaque unité $[(CH_2)_x\text{-}C{=}C\text{-}(CH_2)_y]_p$ et indépendamment pour chaque unité $[(CH_2)_a\text{-}C{=}C\text{-}(CH_2)_b]_r$, un nombre entier compris entre 0 et 12, avantageusement entre 0 et 10, de préférence entre 0 et 9, en particulier entre 2 et 9, et plus particulièrement entre 3 et 8;
$R^1$, $R^2$, $R^3$, $R^4$ et $R^5$ sont un hydrogène ;
p est 1, 2 ou 3 indépendamment pour chaque unité $[(CH_2)_x\text{-}C{=}C\text{-}(CH_2)_y]_p$;
r est 1, 2 ou 3 indépendamment pour chaque unité $[(CH_2)_a\text{-}C{=}C\text{-}(CH_2)_b]_r$;
n est 1.

Selon un mode de réalisation particulier de la présente invention, ladite composition A peut consister en un ou plusieurs composés de formule (I) ou (II).

Dans le procédé selon la présente invention, l'amine tertiaire peut être de formule $NR^8R^9R^{10}$ dans laquelle $R^8$, $R^9$ et $R^{10}$ représentent indépendamment les uns des autres un alkyle en $C_1$-$C_{10}$, ou $R^8$ et $R^9$ forment ensemble avec l'atome d'azote auquel ils sont attachés un hétérocycle à quatre, cinq ou six chaînons. Avantageusement, l'amine tertiaire peut être de formule $NR^8R^9R^{10}$ dans laquelle $R^8$, $R^9$ et $R^{10}$ représentent indépendamment les uns des autres un alkyle en $C_1$-$C_{10}$, ou $R^8$ et $R^9$ forment ensemble avec l'atome d'azote auquel ils sont attachés un hétérocycle choisi parmi le groupe consistant en azetidine, diazetidine, pyrrolidine, imidazolidine, pyrazolidine, piperidine et piperazine. Les hétérocycles imidazolidine et pyrazolidine comprennent deux atomes d'azote. Dans le cadre de la présente invention, les deux atomes d'azote de ces hétérocycles imidazolidine et pyrazolidine sont tertiaires. De préférence, l'amine tertiaire peut être de formule $NR^8R^9R^{10}$ dans laquelle $R^8$, $R^9$ et $R^{10}$ représentent indépendamment les uns des autres alkyle en $C_1$-$C_{10}$, ou $R^8$ et $R^9$ forment ensemble avec l'atome d'azote auquel ils sont attachés une pyrrolidine ou une pipéridine. En particulier, l'amine tertiaire peut être de formule $NR^8R^9R^{10}$ dans laquelle $R^8$, $R^9$ et $R^{10}$ représentent indépendamment les uns des autres un alkyle en $C_1$-$C_6$.

L'amine tertiaire peut être supportée sur une résine. Ainsi, le substituant $R^{10}$ peut être un groupement espaceur entre l'atome d'azote de l'amine tertiaire et la résine. $R^{10}$ peut être un alkyle en $C_1$-$C_{10}$, un benzyle, un aryle en $C_6$-$C_{18}$ ou un cycloalkyle en $C_3$-$C_{10}$.

De préférence, ladite amine tertiaire présente un pKa supérieur à 6, avantageusement supérieur à 7, de préférence supérieur à 8, en particulier supérieur à 9. De préférence, ladite amine tertiaire présente un pKa inférieur à 15, avantageusement inférieur à 14, de préférence inférieur à 12, en particulier inférieur à 11. Ladite amine tertiaire peut ainsi présenter un pKa compris entre 6 et 14, avantageusement compris entre 8 et 12, de préférence entre 9 et 11.

Le présent procédé est effectué sous pression d'une atmosphère d'hydrogène et de monoxyde de carbone. La pression se rapporte à la somme des pressions partielles de monoxyde de carbone et d'hydrogène. La pression peut être comprise entre 50 bars et 200 bars, avantageusement entre 65 bars et 150 bars, de préférence entre 70 bars et 130 bars, en particulier entre 75 bars et 120 bars.

De préférence, le rapport molaire entre le monoxyde de carbone et l'hydrogène peut être compris entre 2 : 1 et 1 : 10, avantageusement le rapport molaire entre le monoxyde de carbone et l'hydrogène est compris entre 1 : 1 et 1 : 5, de préférence le rapport molaire est compris entre 1 : 1 et 1 : 3, en particulier le rapport molaire entre le monoxyde de carbone et l'hydrogène est compris entre 1 : 1 et 1 : 2.

De préférence, l'étape a) du présent procédé peut être réalisée à une température comprise entre 60°C et 200°C, avantageusement entre 70°C et 180°C, de préférence entre 80°C et 150°C.

L'étape a) du présent procédé peut être effectuée en présence d'un solvant organique tel que des hydrocarbures aromatiques ou aliphatiques. Par exemple, le solvant peut être le toluène, le benzène, l'hexane ou l'heptane.

Comme mentionné ci-dessus, le procédé selon la présente invention s'effectue en présence d'au moins un précatalyseur. Ledit précatalyseur est un complexe comprenant un métal de transition choisi parmi la colonne 9 du tableau périodique des éléments. Avantageusement, ledit précatalyseur est un complexe comprenant un métal de transition choisi parmi le cobalt ou le rhodium. Avantageusement, le précatalyseur est un complexe comprenant du rhodium comme métal de transition et un ou plusieurs ligands. De préférence, au moins un desdits un ou plusieurs ligands est choisi parmi CO, acetylacetonate, cyclooctadiene, norbornène, acétate. Le précatalyseur peut être supporté

sur un support solide. Le support peut être le noir de carbone, $SiO_2$, $Al_2O_3$, $TiO_2$, MgO, ZnO, $CaCO_3$, $CaSO_4$ ou $MgSO_4$ ou une combinaison de ceux-ci. Le rapport en poids entre le support et le précatalyseur peut être compris entre 1 et 100.

De préférence, le rapport molaire entre l'amine tertiaire de formule $NR^8R^9R^{10}$ telle que décrite ci-dessus et le métal de transition du complexe utilisé comme précatalyseur à l'étape a) du présent procédé est supérieur à 20, avantageusement supérieur à 50, de préférence supérieur à 100, en particulier supérieur à 200.

Lesdits un ou plusieurs ligands peuvent également comprendre au moins un ligand phosphoré monodentate ou bidentate comprenant au moins un substituant aryle en $C_6$-$C_{18}$ substitué en position ortho par rapport à l'atome de phosphore ou comprenant au moins un substituant aryloxy en $C_6$-$C_{18}$. Avantageusement, ledit ligand phosphoré peut être de formule $P(Ar)_3$ ou $(Ar)_2$-P-L-$P(Ar)_2$ dont laquelle Ar est un groupement aryle en $C_6$-$C_{18}$ substitué au moins en position ortho par rapport à l'atome de phosphore par un groupement sélectionné parmi le groupe consistant en $C_1$-$C_6$ alkyle, phényle, benzyle, $C_3$-$C_6$ cycloalkyle, halogène, alcoxyle en $C_1$-$C_6$, aryloxy en $C_6$; et L est un bras espaceur sélectionné parmi le groupe consistant en alkyle en $C_1$-$C_6$, aryle en $C_6$-$C_{12}$, cycloalkyle en $C_3$-$C_{10}$. L'expression « substitué en position ortho par rapport à l'atome de phosphore » signifie que, dans le cycle aryle, au moins une des deux positions ortho par rapport à l'atome de carbone lié à l'atome de phosphore est substituée par l'un de groupements mentionnés. De préférence, ledit ligand phosphoré peut être de formule $P(Ar)_3$ dans laquelle Ar est un groupement aryle en $C_6$-$C_{18}$ substitué en position ortho par rapport à l'atome de phosphore par un groupement sélectionné parmi le groupe consistant en méthyle, éthyle, méthoxy, phényle, benzyle, -F, cyclohexyle.

Alternativement, ledit ligand phosphoré peut être de formule $P(O\text{-}Ar)_3$ ou $(Ar\text{-}O)_2$-P-L-$P(O\text{-}Ar)_2$ dans laquelle Ar est un groupement aryle en $C_6$-$C_{18}$ substitué ou non par groupement sélectionné parmi le groupe consistant en $C_1$-$C_6$ alkyle, $C_3$-$C_6$ alkyle, halogène, alcoxyle en $C_1$-$C_6$, aryloxy en $C_6$ ; et L est un bras espaceur sélectionné parmi le groupe consistant en alkyle en $C_1$-$C_6$, aryle en $C_6$-$C_{12}$, cycloalkyle en $C_3$-$C_{10}$. De préférence, ledit ligand phosphoré peut être de formule $P(O\text{-}Ar)_3$ dans laquelle Ar est un aryle en $C_6$ substitué ou non un groupement méthyle, éthyle, méthoxy, phényle, benzyle, cyclohexyle.

En particulier, ledit ligand phosphoré peut être $P(OPh)_3$, $P(C_6F_5)_3$, $P(o\text{-}MePh)_3$, $P(o\text{-}OMePh)_3$.

Alternativement, lesdits un ou plusieurs ligands comprennent également au moins un ligand phosphoré monodentate ou bidentate hydrosoluble comprenant au moins un groupement fonctionnel $SO_3^-X^+$, $NR_3^+A^-$, $CO_2^-X^+$, X représentant Li, Na ou K ; et A représentant Cl, Br ou I. Avantageusement, ledit ligand phosphoré peut être de formule $P(Ar)_3$ ou $(Ar)_2$-P-L-$P(Ar)_2$ dans laquelle L est un bras espaceur sélectionné parmi le groupe consistant en alkyle en $C_1$-$C_6$, aryle en $C_6$-$C_{12}$, cycloalkyle en $C_3$-$C_{10}$ ; et Ar est un aryle en $C_6$-$C_{18}$ substitué par au moins un groupement fonctionnel $SO_3^-X^+$, $NR_3^+A^-$, $CO_2^-X^+$, X représentant Li, Na ou K, et A représentant Cl, Br ou I ; et optionnellement substitué par un groupement sélectionné parmi le groupe consistant en $C_1$-$C_6$ alkyle, phényle, benzyle, $C_3$-$C_6$ cycloalkyle, halogène, alcoxyle en $C_1$-$C_6$, aryloxy en $C_6$.

Selon un mode de réalisation particulier, l'étape a) met également en présence une cyclodextrine -$\alpha$, -$\beta$, $\gamma$-méthylée présentant un degré de substitution moyen compris entre 0,5 et 2,0 ou une cyclodextrine -$\alpha$, -$\beta$, -$\gamma$ hydroxylée présentant un degré de substitution moyen compris entre 0,5 et 0,9. Ladite cyclodextrine -$\alpha$, -$\beta$, -$\gamma$ méthylée peut présenter un degré de substitution moyen compris entre 1,6 et 2,0, ou compris entre 0,9 et 1,6 ou compris entre 0,5 et 0,9. Lorsqu'une cyclodextrine et un ligand phosphoré hydrosoluble tel que mentionnée ci-dessus sont utilisés à l'étape a), de l'eau est également ajouté à cette même étape a) pour créer un milieu biphasique comprenant une phase organique et une phase aqueuse. Avant la mise en oeuvre de la réaction, la phase organique est notamment constituée du composé de formule (I) ou (II) selon la présente invention et de l'amine tertiaire. Dans ce mode de réalisation, l'amine tertiaire est de formule $NR^8R^9R^{10}$ dans laquelle $R^8$, $R^9$ et $R^{10}$ représentent indépendamment l'un de l'autre un alkyle en $C_4$-$C_{10}$. Lorsque le procédé est réalisé en présence d'une cyclodextrine, le précatalyseur comprenant au moins un ligand phosphoré monodentate ou bidentate hydrosoluble telle que défini ci-dessus. En outre, le procédé est alors réalisé dans des conditions opératoires permettant l'établissement d'une émulsion lors de l'agitation et une décantation des produits de la réaction après arrêt de l'agitation, de préférence la décantation d'au moins une partie du composé selon la présente invention. Avantageusement, la proportion de cyclodextrine est comprise entre 15 et 40% en poids sur base du poids total d'eau, de cyclodextrine et desdits un ou plusieurs composés de formule (I), de préférence de formule (II) telle que décrite ci-dessus, mis en présence à l'étape a).

Le procédé selon la présente invention peut également comprendre une étape de recyclage lorsque le procédé est effectué en milieu biphasique. L'étape de recyclage comprend le dégazage du réacteur dans lequel le procédé selon la présente invention est effectué, le prélèvement de la phase organique sous atmosphère contrôlée, et l'ajout dans le réacteur de la composition A comprenant un ou plusieurs composés de formule (I) ou de formule (II) telles que décrites ci-dessus, de l'amine tertiaire $NR^8R^9R^{10}$ et optionnellement d'un ou plusieurs ligands tels que décrits ci-dessus. Lors de cette étape de recyclage, la température peut rester constante, c'est-à-dire rester à la température à laquelle la réaction d'hydrohydroxyméthylation a été effectuée, ou être à une température supérieure ou inférieure à la température à laquelle la réaction d'hydrohydroxyméthylation a été effectuée.

Selon un mode de réalisation particulier de la présente invention, ladite composition A comprenant un ou plusieurs composés de formule (I), de préférence comprenant un ou plusieurs composés de formule (II), est une huile

végétale ayant un nombre moyen d'insaturations compris entre 0,5 et 20, avantageusement entre 0,5 et 15, de préférence entre 0,5 et 10.

De préférence, ladite composition A comprenant un ou plusieurs composés de formule (I), de préférence de formule (II), est une huile végétale ayant un nombre moyen d'insaturations inférieur à 3,5, avantageusement inférieur à 3,4, de préférence inférieur à 3,3, plus préférentiellement inférieur à 3,2, en particulier inférieur à 3,1, plus particulièrement inférieur à 3,0. De préférence, ladite huile végétale a un nombre moyen d'insaturations supérieur à 0,5, avantageusement supérieur à 1, de préférence supérieur à 1,5, plus préférentiellement supérieur à 2,0, en particulier supérieur à 2,5. Ladite huile végétale peut ainsi avoir un nombre moyen d'insaturations compris entre 2,0 et 3,2, avantageusement entre 2,4 et 3,1, de préférence entre 2,5 et 3,0, en particulier entre 2,6 et 2,8.

Alternativement, ladite composition A comprenant un ou plusieurs composés de formule (I), de préférence de formule (II), est une huile végétale ayant un nombre moyen d'insaturations compris entre 4 et 10.

## Méthodes

### Détermination du nombre moyen d'insaturations

Le nombre moyen d'insaturations est déterminé par $^1$H RMN dans le chloroforme deutéré ($CDCl_3$). Les analyses RMN sont effectuées sur un appareil BRUKER 300 MHz. Le nombre moyen d'insaturations d'un composé est calculé à partir de la valeur d'intégration du signal $^1$H RMN des protons oléfiniques situés sur le ou les motifs acides gras du composé. Le signal en $^1$H RMN des protons oléfiniques se situe à 5.3 ppm. Si le signal des protons oléfiniques est recouvert par un ou plusieurs autres protons, un facteur de normalisation est appliqué pour déduire la contribution de ce(s) dernier(s) proton(s) dans la valeur d'intégration et ainsi obtenir une valeur d'intégration correspondant uniquement aux protons oléfiniques.

Dans le cas des composés de formule (II) où $R^3$ est un hydrogène dans l'une quelconque des unités n, le signal de cet atome d'hydrogène en $^1$H RMN recouvre le signal des protons oléfiniques. Un facteur de normalisation, noté FN, est ainsi calculé selon l'équation :

FN = (valeur de l'intégration d'un signal / nombre de protons théorique du signal correspondant)

Par exemple, pour le cas des triglycérides, le facteur de normalisation est calculé à partir de la valeur d'intégration du signal $^1$H RMN correspondant aux protons des deux groupements $CH_2$ du glycérol selon l'équation FN = B/4. La figure 2 représente le spectre $^1$H RMN de la trioléine. Le signal B représentatif du signal correspondant aux protons des deux groupements $CH_2$ du motif glycérol a pour valeur 4 et le facteur de normalisation est donc de 1. Le proton CH du glycérol recouvre le signal des protons oléfiniques. Le nombre moyen d'insaturations est donc déduit de l'équation suivante :

$$DBi = (A - FN) / 2$$

où A correspond à la valeur d'intégration du signal correspondant au proton CH du glycérol et des protons oléfiniques (comme indiqué à la figure 2), et FN est le facteur de normalisation décrit ci-dessus.

Lorsque le composé analysé n'est pas un triglycéride, l'homme du métier adaptera le calcul du facteur de normalisation FN sur base d'un autre proton ou d'un autre groupe de proton, par exemple les protons situés en position C ou D sur la figure 2.

### Conversion de la réaction

La conversion de la réaction est donnée par la formule suivante :

$$Conv. (\%) = ((DBi - DBf) / DBi)*100 = ((Ai - Af) / (Ai - FN))*100$$

où DBi et DBf sont respectivement le nombre d'insaturations initiales et finales et Ai et Af l'intégration des signaux A en début et fin de réaction. Les signaux A correspondent aux signaux en $^1$H RMN des protons oléfiniques tel qu'expliqué ci-dessus. Si le signal des protons oléfiniques est recouvert par un ou plusieurs autres protons ne correspondant pas aux protons oléfiniques, un facteur de normalisation FN est appliqué et calculé comme expliqué ci-dessus.

**Détermination de la sélectivité en alcool**

Les sélectivités en aldéhydes, alcools et C=C hydrogénées supportées par les produits issus de la réaction sont déterminées par intégration des signaux $^1H$ RMN. La figure 3 représente un spectre $^1H$ RMN du produit issu de la mise en oeuvre du procédé selon l'invention à partir de la trioléine. Le signal des protons $CH_2$ du groupement hydroxyméthylé (noté C à la figure 3) se situe à 3,54 ppm. La sélectivité en alcool de la réaction est donnée par la formule :

$$\text{Selec. (HHM) (\%)} = ((C / (2^*FN)) / (DBi - DBf))^*100$$

où C représente la valeur d'intégration du signal correspondant aux protons $CH_2$ du groupement hydroxyméthylé, FN représente le facteur de normalisation, DBi et DBf représentent le nombre de doubles liaisons initiales et finales.

**Exemples**

**Exemple 1**

Dans un réacteur, 1 mL de trioléine (1 mmol), 5 mL de toluène, 3,9 mg de $Rh(CO)_2(acac)$ (0,015 mmol), 3 mmol d'amine sont mélangés. La réaction est effectuée sous une pression de 80 bars de monoxyde de carbone et d'hydrogène (rapport molaire 1:1) pendant 18 heures à 80°C.

Le tableau 1 ci-dessous reporte les résultats obtenus avec différentes amines tertiaires.

*Tableau 1 - Performances catalytiques en fonction de l'amine tertiaire*

| N° | amine | Conversion (%) | Selec. Aldéhyde (%) | Selec. Alcool (%) | Selec. Hydrogénation (%) |
|---|---|---|---|---|---|
| **1** | $N(Et)_3$ | 100 | 0 | 93 | 7 |
| **2** | $N(Bu)_3$ | 100 | 0 | 96 | 4 |
| **3** | $N(Hex)_3$ | 100 | 0 | 95 | 5 |
| **4** | N-Me pyrolidine | 100 | 0 | 95 | 5 |
| **5** | TMEDA | 100 | 8 | 82 | 9 |
| **6** | NMP | 100 | 85 | 0 | 15 |
| **7** | N-Me Pyrrole | 100 | 75 | 0 | 25 |
| **8** | Pyridine | 100 | 78 | 0 | 22 |
| **9** | 1,10-phen | 100 | 76 | 0 | 24 |
| *Conditions de réaction : trioléine : 1 mmol (1 mL, 3 mmol de C=C) ; $Rh(CO)_2acac$ : 0.015 mmol (3.9 mg) ; toluène : 5ml ; aminé : 3 mmol (TMEDA et 1, 10-phen : 1.5 mmol) ; pression $CO/H_2$ (1 :1) : 80 bar; température : 80 °C ; 18 heures TMEDA :N, N, N', N'-tetramethylethylenediamine ; NMP : N-methyl-2-pyrrolidone; 1,10-phen : 1,10-phenanthroline* | | | | | |

Les résultats de l'exemple 1 démontre que l'utilisation d'amine tertiaire permet la synthèse de polyols « one pot » sans utilisation de co-catalyseur ou autres additifs dans des conditions catalytiques relativement douces. L'utilisation d'amines tertiaires aliphatiques, cycliques ou non, permet la formation majoritaire des polyols souhaités avec une excellente conversion et sélectivité. Cependant, l'utilisation d'hétéroaryles azotés (pyridine et pyrrole) ou d'amides mène à une réaction d'hydroformylation sans étape d'hydrogénation donnant donc en majorité des aldéhydes.

**Exemple 2**

L'exemple 1 a été reproduit en utilisant comme amine tertiaire la tributylamine et en faisant varier la pression en monoxyde de carbone et en hydrogène. La durée de la réaction est de 6 heures. Le tableau 2 reprend les résultats obtenus.

*Tableau 2 - Performances catalytiques en fonction de la pression en gaz de synthèse*

| N° | Pression | Conversion (%) | Selec. Aldéhyde (%) | Selec. Alcool (%) | Selec. Hydrogénation (%) |
|---|---|---|---|---|---|
| **10** | 80 | 91 | 39 | 53 | 8 |
| **11** | 100 | 100 | 20 | 75 | 5 |
| *Conditions de réaction : trioléine : 1 mmol (1mL, 3 mmol de C=C) ; $Rh(CO)_2acac$ : 0.015 mmol (3.9mg) ; toluène : 5ml ; tributylamine: 3 mmol ; $CO/H_2$ (1 :1) ; température : 80°C ;* 6 *heures* | | | | | |

La pression totale en gaz de synthèse $CO/H_2$ a une influence sur les conversions d'hydrohydroxyméthylation ainsi que sur les sélectivités en produits finaux. Lorsque la pression totale augmente, la conversion et la sélectivité en alcool augmentent également. Il est également à noter que plus la pression augmente, plus la sélectivité en hydrogénation des C=C diminue. Cependant en augmentant sensiblement la pression, la première étape d'hydroformylation est favorisée, suivie de l'étape d'hydrogénation des aldéhydes nouvellement formés, l'hydrogénation du substrat devant alors être plus lente.

## Exemple 3

L'exemple 1 a été reproduit en présence de tributylamine pendant 6 heures en faisant varier la température de réaction. Le tableau 3 reprend les résultats obtenus.

*Tableau 3 - Performances catalytiques en fonction de la température*

| N° | Température (°C) | Conversion (%) | Selec. Aldéhyde (%) | Selec. Alcool (%) | Selec. Hydrogénation (%) |
|---|---|---|---|---|---|
| **12** | 80 | 91 | 39 | 53 | 8 |
| **13** | 110 | 99 | 2 | 89 | 9 |
| **14** | 140 | 100 | 0 | 93 | 7 |
| *Conditions de réaction : trioléine : 1 mmol (1mL, 3 mmol de C=C) ; $Rh(CO)_2acac$ : 0.015 mmol (3.9mg) ; toluène : 5ml ; tributylamine: 3 mmol ; pression $CO/H_2$ (1 :1) : 80 bar ; 6 heures* | | | | | |

Au-dessus de 80°C, la conversion des triglycérides augmente, s'accompagnant d'une forte activité en hydrogénation des aldéhydes produits et limitant de façon surprenant l'hydrogénation des doubles liaisons carbone-carbone du substrat.

## Exemple 4

L'exemple 4 vise à déterminer l'influence du rapport molaire $CO/H_2$ sur la réaction d'hydrohydroxyméthylation. L'exemple 1 a été reproduit avec la triéthylamine comme amine tertiaire. La durée de la réaction était établie à 6 heures. Les résultats sont repris dans le tableau 4 ci-dessous.

*Tableau 4. Influence du rapport $CO/H_2$*

| N° | Rapport CO/H2 | Amine | Conversion (%) | Selec. Aldéhyde (%) | Selec. Alcool (%) | Selec. Hydrogénation (%) |
|---|---|---|---|---|---|---|
| **15** | 1 : 1 | $N(Et)_3$ | 94 | 12 | 81 | 7 |
| **16** | 1 : 2 | $N(Et)_3$ | 95 | 1 | 93 | 6 |
| *Conditions de réaction : trioléine : 1 mmol (1mL, 3 mmol de C=C) ; $Rh(CO)_2acac$ : 0.015 mmol (3.9 mg) ; toluène : 5ml ; aminé : 3 mmol ; pression totale $CO/H_2$: 80 bar ; Température : 80°C ; 6 heures* | | | | | | |

L'augmentation de la pression partielle d'hydrogène a un effet surprenant sur la formation d'alcool. L'utilisation d'un rapport molaire 1 : 2 en CO/H2 permet d'atteindre des proportions stoechiométriques de réaction (une molécule de CO utilisée pour deux molécules d'hydrogène) et rend le système catalytique plus hydrogénant pour parvenir rapidement aux polyols attendus.

**Exemple 5**

Le présent procédé de préparation de polyols a été appliqué à partir d'une huile végétale naturelle. Le tableau 5 reprend les résultats obtenus.

*Tableau 5. Application aux huiles végétales*

| N° | Huiles | Conv. (%) | Nombre moyen d'insaturations | Selec. Aldéhyde (%) | Selec. Alcool (%) | Selec. Hydrogénation (%) | Selec. isomérisation (%) |
|---|---|---|---|---|---|---|---|
| **17** | Olive | 94 | 2.78 | 2 | 90 | 8 | 0 |
| *Conditions de réaction : huile : 1 mmol (1 mL) ; $Rh(CO)_2acac$ : 0.015 mmol (3.9 mg) ; toluène : 5 ml ; triéthylamine: 3 mmol ; pression $CO/H_2$ (1 :1) : 80 bar ; Température : 80 °C* | | | | | | | |

Il ressort de ces résultats que le procédé selon l'invention s'applique aux huiles végétales c'est-à-dire à des compositions comprenant des triglycérides de structure différentes.

**Exemple 6**

L'exemple 1 a été reproduit en présence d'un système auto-émulsifiant. L'addition de cyclodextrine, et notamment de CRYSMEB® (CDs méthylées en position 2 et possédant un dégrée de substitution par unité glucosidique (DS) de 0.8) permet de former un complexe tensioactif à l'interface huile/eau. Ce complexe stabilisant l'interface permet de mettre le système biphasique en émulsion et d'augmenter l'interface entre les deux milieux. Le procédé est réalisé en présence d'un précatalyseur comprenant également un ligand de type phosphine hydrosoluble telle que le sel trisodique de la tripénylphosphine tri sulfonée en position meta (TPPTS). Les résultats sont repris dans le tableau 6 ci-dessous.

*Tableau 6 - Procédé en milieu auto-émulsifiant*

| N° | amine | Conversion (%) | Selec. Aldéhyde (%) | Selec. Alcool (%) | Selec. Hydrogénation (%) |
|---|---|---|---|---|---|
| **18** | $N(Bu)_3$ | 92 | 3 | 92 | 4 |
| **19** | $N(Oct)_3$ | 94 | 3 | 91 | 5 |
| *Conditions de réaction : trioléine : 1 mmol (1 mL, 3 mmol de C=C) ; $Rh(CO)_2acac$ : 0.015 mmol (3.9 mg) ; TPPTS : 0.075 mmol (42 mg), ; eau : 8 ml ; CRYSMEB : 2mmol (2.3 g) ; aminé : 3 mmol ; pression $CO/H_2$ (1 :1) : 80 bar ; Température : 80 °C ; 18 heures.* | | | | | |

L'utilisation de cyclodextrine dans un milieu auto-émulsifiant permet de convertir de façon efficace les insaturations C=C initiales en fonction alcool et ce avec de très bonne sélectivité et en limitant la formation de produit d'hydrogénation.

**Revendications**

1. Procédé de préparation de polyols à partir d'une composition A comprenant un ou plusieurs composés de formule (I)

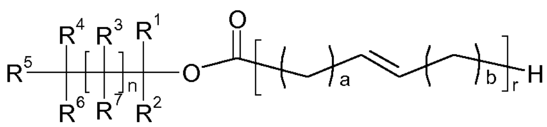

(I)

dans laquelle

$R^1$, $R^2$, $R^3$, $R^4$, $R^5$, $R^6$ et $R^7$ sont indépendamment les uns des autres, et pour $R^3$ et $R^7$ indépendamment pour chacune des unités n, choisis parmi le groupe consistant en H, $-OR^{15}$, alkyle en $C_1$-$C_{10}$ substitué ou non par un ou plusieurs groupes $-OR^{15}$, aryle en $C_6$-$C_{12}$ substitué ou non par un ou plusieurs groupes $-OR^{15}$, ou

cycloalkyle en $C_3$-$C_{10}$ substitué ou non par un ou plusieurs groupes -$OR^{15}$, ou un groupement de formule (Ia)

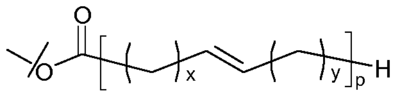

(Ia)

$R^{15}$ représente H ou alkyle en $C_1$-$C_{10}$ substitué ou non par un ou plusieurs groupes -OH a, b, x et y sont indépendamment les uns des autres, indépendamment pour chaque groupement de formule (Ia), indépendamment pour chaque unité $[(CH_2)_a\text{-}C{=}C\text{-}(CH_2)_b]_r$ et indépendamment pour chaque unité $[(CH_2)_x\text{-}C{=}C\text{-}(CH_2)_y]_p$, un nombre entier compris entre 0 et 20, avantageusement entre 0 et 15, de préférence entre 0 et 12 ;
r est un nombre entier compris entre 1 et 10, avantageusement entre 1 et 5 ;
p est un nombre entier compris entre 1 et 10, avantageusement entre 1 et 5 ;
n est un nombre entier entre 1 et 7 ;
ledit procédé comprenant une étape a) de mise en présence sous agitation et sous une atmosphère d'hydrogène et de monoxyde de carbone :

- d'au moins un précatalyseur étant un complexe comprenant un métal de transition choisi parmi la colonne 9 du tableau périodique,
- d'une amine tertiaire, ou un sel d'ammonium non quaternaire de celle-ci, de formule $NR^8R^9R^{10}$ dans laquelle $R^8$, $R^9$ et $R^{10}$ représentent indépendamment les uns des autres un alkyle en $C_1$-$C_{10}$, aryle en $C_6$-$C_{12}$, cycloalkyle en $C_3$-$C_{10}$ ou $R^8$ et $R^9$ forment ensemble avec l'atome d'azote auquel ils sont attachés un hétérocycle à quatre, cinq ou six chaînons,
- de ladite composition A comprenant ledit composé de formule (I).

**2.** Procédé selon l'une quelconque des revendications précédentes **caractérisé en ce que** l'étape a) est réalisée à une température comprise entre 70°C et 180°C, de préférence entre 80°C et 150°C.

**3.** Procédé selon l'une quelconque des revendications précédentes **caractérisé en ce que** ledit au moins un composé de formule (I) est de formule (II)

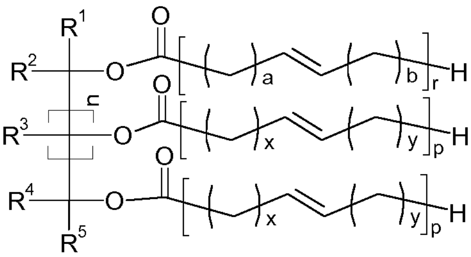

(II)

dans laquelle

a, b, x et y sont indépendamment les uns des autres, indépendamment pour chaque groupement de formule (Ia), indépendamment pour chaque unité $[(CH_2)_a\text{-}C{=}C\text{-}(CH_2)_b]_r$ et indépendamment pour chaque unité $[(CH_2)_x\text{-}C{=}C\text{-}(CH_2)_y]_p$, un nombre entier compris entre 0 et 20, avantageusement entre 0 et 15, de préférence entre 0 et 12 ;
p est indépendamment pour chaque unité $[(CH_2)_x\text{-}C{=}C\text{-}(CH_2)_y]_p$ un nombre entier compris entre 1 et 5 ;
r est indépendamment pour chaque unité $[(CH_2)_a\text{-}C{=}C\text{-}(CH_2)_b]_r$ un nombre entier compris entre 1 et 5 ;
$R^1$, $R^2$, $R^3$, $R^4$ et $R^5$ sont indépendamment les uns des autres, et pour $R^3$ indépendamment pour chacune des unités n, choisis parmi le groupe consistant en H, -$OR^{15}$, alkyle en $C_1$-$C_{10}$ substitué ou non par un ou plusieurs groupes -$OR^{15}$, ou un groupement de formule (Ia) tel que décrit ci-dessus,
$R^{15}$ représente H ou alkyle en $C_1$-$C_{10}$ substitué ou non par un ou plusieurs groupes -OH ;
n est un nombre entier entre 1 et 4.

**4.** Procédé selon la revendication précédente **caractérisé en ce que** ledit au moins un composé de formule (I) est de

formule (II) dans laquelle

a et x peuvent être indépendamment l'un de l'autre, indépendamment pour chaque unité $[(CH_2)_x\text{-}C{=}C\text{-}(CH_2)_y]_p$ et indépendamment pour chaque unité $[(CH_2)_a\text{-}C{=}C\text{-}(CH_2)_b]_r$, un nombre entier compris entre 1 et 12, avantageusement entre 2 et 10, de préférence entre 3 et 9, en particulier entre 3 et 8 et plus particulièrement entre 4 et 8 ;
b et y peuvent être indépendamment l'un de l'autre, indépendamment pour chaque unité $[(CH_2)_x\text{-}C{=}C\text{-}(CH_2)_y]_p$ et indépendamment pour chaque unité $[(CH_2)_a\text{-}C{=}C\text{-}(CH_2)_b]_r$, un nombre entier compris entre 0 et 12, avantageusement entre 0 et 10, de préférence entre 0 et 9, en particulier entre 2 et 9, et plus particulièrement entre 3 et 8;
$R^1$, $R^2$, $R^3$, $R^4$ et $R^5$ sont un hydrogène ;
p est 1, 2 ou 3 indépendamment pour chaque unité $[(CH_2)_x\text{-}C{=}C\text{-}(CH_2)_y]_p$;
r est 1, 2 ou 3 indépendamment pour chaque unité $[(CH_2)_a\text{-}C{=}C\text{-}(CH_2)_b]_r$;
n est 1.

5. Procédé selon l'une quelconque des revendications précédentes **caractérisé en ce que** l'amine tertiaire est de formule $NR^8R^9R^{10}$ dans laquelle $R^8$, $R^9$ et $R^{10}$ représentent indépendamment les uns des autres un alkyle en $C_1$-$C_{10}$, ou $R^8$ et $R^9$ forment ensemble avec l'atome d'azote auquel ils sont attachés un hétérocycle azetidine, diazetidine, pyrrolidine, imidazolidine ou pyrazolidine ; de préférence $R^8$ et $R^9$ forment ensemble avec l'atome d'azote auquel ils sont attachés une pyrrolidine.

6. Procédé selon l'une quelconque des revendications précédentes, **caractérisé en ce que** le précatalyseur est un complexe comprenant du rhodium comme métal de transition et un ou plusieurs ligands ; de préférence au moins un desdits un ou plusieurs ligands est choisi parmi CO, acetylacetonate, cyclooctadiene, norbornène et acétate.

7. Procédé selon l'une quelconque des revendications précédentes **caractérisé en ce qu'**il est effectué sous pression d'une atmosphère d'hydrogène et de monoxyde de carbone, ladite pression étant comprise entre 50 bars et 200 bars, avantageusement entre 65 bars et 150 bars, de préférence entre 70 bars et 130 bars, en particulier entre 75 bars et 120 bars.

8. Procédé selon l'une quelconque des revendications précédentes **caractérisé en ce que** le rapport molaire entre le monoxyde de carbone et l'hydrogène est compris entre 2 : 1 et 1 : 10, avantageusement le rapport molaire entre le monoxyde de carbone et l'hydrogène est compris entre 1 : 1 et 1 : 5, de préférence le rapport molaire est compris entre 1 : 1 et 1 : 3, en particulier le rapport molaire entre le monoxyde de carbone et l'hydrogène est compris entre 1 : 1 et 1 : 2.

9. Procédé selon l'une quelconque des revendications précédentes **caractérisé en ce que** le rapport molaire entre l'amine et le métal de transition est supérieur à 20, avantageusement supérieur à 50, de préférence supérieur à 100, en particulier supérieur à 200.

10. Procédé selon l'une quelconque des revendications précédentes **caractérisé en ce que** ladite composition A comprenant un ou plusieurs composés de formule (I) est une huile végétale ayant un nombre moyen d'insaturations inférieur à 3,5, avantageusement inférieur à 3,4, de préférence inférieur à 3,3, plus préférentiellement inférieur à 3,2, en particulier inférieur à 3,1, plus particulièrement inférieur à 3,0.

**Patentansprüche**

1. Verfahren zur Herstellung von Polyolen aus einer Zusammensetzung A, die eine oder mehrere Verbindungen der Formel (I)

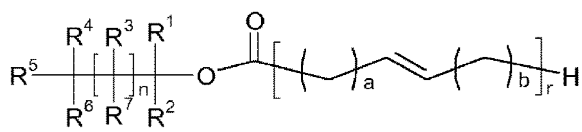

(I)

umfasst, wobei

$R^1$ , $R^2$, $R^3$, $R^4$, $R^5$, $R^6$ und $R^7$ unabhängig voneinander und für $R^3$ und $R^7$ unabhängig für jede der n Einheiten aus der Gruppe bestehend aus H, $-OR^{15}$, $C_1$-$C_{10}$-Alkyl, das gegebenenfalls durch eine oder mehrere $-OR^{15}$-Gruppen substituiert ist, $C_6$-$C_{12}$-Aryl, das gegebenenfalls durch eine oder mehrere $-OR^{15}$-Gruppen substituiert ist, oder $C_3$-$C_{10}$-Cycloalkyl, das gegebenenfalls durch eine oder mehrere $-OR^{15}$-Gruppen substituiert ist, oder eine Gruppe der Formel (Ia)

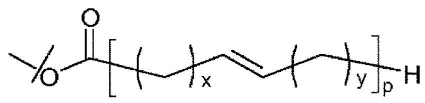

(Ia)

ausgewählt sind;
$R^{15}$ für H oder $C_1$-$C_{10}$-Alkyl, das gegebenenfalls durch eine oder mehrere -OH-Gruppen substituiert ist, steht;
a, b, x und y unabhängig voneinander, unabhängig für jede Gruppe der Formel (Ia), unabhängig für jede $[(CH_2)_a\text{-}C{=}C\text{-}(CH_2)_b]_r$-Einheit und unabhängig für jede $[(CH_2)_x\text{-}C{=}C\text{-}(CH_2)_y]_p$-Einheit eine ganze Zahl zwischen 0 und 20, vorteilhafterweise zwischen 0 und 15, vorzugsweise zwischen 0 und 12, sind;
r eine ganze Zahl zwischen 1 und 10, vorteilhafterweise zwischen 1 und 5, ist;
p eine ganze Zahl zwischen 1 und 10, vorteilhafterweise zwischen 1 und 5, ist;
n eine ganze Zahl zwischen 1 und 7 ist;
wobei das Verfahren einen Schritt a) umfasst, bei dem man unter Rühren und unter Wasserstoff- und Kohlenmonoxid-Atmosphäre:

- mindestens einen Präkatalysator, bei dem es sich um einen Komplex handelt, der ein Übergangsmetall der Spalte 9 des Periodensystems umfasst,
- ein tertiäres Amin, oder ein nichtquaternäres Ammoniumsalz davon, der Formel $NR^8R^9R^{10}$, wobei $R^8$, $R^9$ und $R^{10}$ unabhängig voneinander für ein $C_1$-$C_{10}$-Alkyl, ein $C_6$-$C_{12}$-Aryl oder ein $C_3$-$C_{10}$-Cycloalkyl stehen oder $R^8$ und $R^9$ zusammen mit dem Stickstoffatom, an das sie gebunden sind, einen vier-, fünf- oder sechsgliedrigen Heterocyclus bilden,
- die Zusammensetzung A, die die Verbindung der Formel (I) umfasst,

zusammenbringt.

2. Verfahren nach einem der vorhergehenden Ansprüche, **dadurch gekennzeichnet, dass** der Schritt a) bei einer Temperatur zwischen 70 °C und 180 °C, vorzugsweise zwischen 80 °C und 150 °C, durchgeführt wird.

3. Verfahren nach einem der vorhergehenden Ansprüche, **dadurch gekennzeichnet, dass** die mindestens eine Verbindung der Formel (I) die Formel (II)

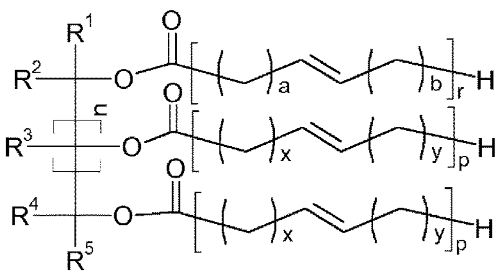

(II)

aufweist, wobei

a, b, x und y unabhängig voneinander, unabhängig für jede Gruppe der Formel (Ia), unabhängig für jede $[(CH_2)_a\text{-}C{=}C\text{-}(CH_2)_b]_r$-Einheit und unabhängig für jede $[(CH_2)_x\text{-}C{=}C\text{-}(CH_2)_y]_p$-Einheit eine ganze Zahl zwischen 0 und 20, vorteilhafterweise zwischen 0 und 15, vorzugsweise zwischen 0 und 12, sind;

p unabhängig für jede $[(CH_2)_x\text{-}C{=}C\text{-}(CH_2)_y]_p$-Einheit eine ganze Zahl zwischen 1 und 5 ist;
r unabhängig für jede $[(CH_2)_a\text{-}C{=}C\text{-}(CH_2)_b]_r$-Einheit eine ganze Zahl zwischen 1 und 5 ist;
$R^1$, $R^2$, $R^3$, $R^4$ und $R^5$ unabhängig voneinander und für $R^3$ unabhängig für jede der n Einheiten aus der Gruppe bestehend aus H, $-OR^{15}$, $C_1$-$C_{10}$-Alkyl, das gegebenenfalls durch eine oder mehrere $-OR^{15}$-Gruppen substituiert ist, oder eine Gruppe der Formel (Ia) gemäß obiger Beschreibung ausgewählt sind,
$R^{15}$ für H oder $C_1$-$C_{10}$-Alkyl, das gegebenenfalls durch eine oder mehrere -OH-Gruppen substituiert ist, steht;
n eine ganze Zahl zwischen 1 und 4 ist.

4. Verfahren nach dem vorhergehenden Anspruch, **dadurch gekennzeichnet, dass** die mindestens eine Verbindung der Formel (I) die Formel (II) aufweist, wobei

a und x unabhängig voneinander, unabhängig für jede $[(CH_2)_x\text{-}C{=}C\text{-}(CH_2)_y]_p$-Einheit und unabhängig für jede $[(CH_2)_a\text{-}C{=}C\text{-}(CH_2)_b]_r$-Einheit eine ganze Zahl zwischen 1 und 12, vorteilhafterweise zwischen 2 und 10, vorzugsweise zwischen 3 und 9, speziell zwischen 3 und 8 und spezieller zwischen 4 und 8 sind;
b und y unabhängig voneinander, unabhängig für jede $[(CH_2)_x\text{-}C{=}C\text{-}(CH_2)_y]_p$-Einheit und unabhängig für jede $[(CH_2)_a\text{-}C{=}C\text{-}(CH_2)_b]_r$-Einheit eine ganze Zahl zwischen 0 und 12, vorteilhafterweise zwischen 0 und 10, vorzugsweise zwischen 0 und 9, speziell zwischen 2 und 9 und spezieller zwischen 3 und 8 sind;
$R^1$, $R^2$, $R^3$, $R^4$ und $R^5$ für Wasserstoff stehen;
p unabhängig für jede $[(CH_2)_x\text{-}C{=}C\text{-}(CH_2)_y]_p$-Einheit 1, 2 oder 3 ist;
r unabhängig für jede $[(CH_2)_a\text{-}C{=}C\text{-}(CH_2)_b]_r$-Einheit 1, 2 oder 3 ist;
n 1 ist.

5. Verfahren nach einem der vorhergehenden Ansprüche, **dadurch gekennzeichnet, dass** das tertiäre Amin die Formel $NR^8R^9R^{10}$ aufweist, wobei $R^8$, $R^9$ und $R^{10}$ unabhängig voneinander für ein $C_1$-$C_{10}$-Alkyl stehen oder $R^8$ und $R^9$ zusammen mit dem Stickstoffatom, an das sie gebunden sind, einen Azetidin-, Diazetidin-, Pyrrolidin-, Imidazolidin- oder Pyrazolidin-Heterocyclus bilden; vorzugsweise $R^8$ und $R^9$ zusammen mit dem Stickstoffatom, an das sie gebunden sind, ein Pyrrolidin bilden.

6. Verfahren nach einem der vorhergehenden Ansprüche, **dadurch gekennzeichnet, dass** es sich bei dem Präkatalysator um einen Komplex handelt, der Rhodium als Übergangsmetall und einen oder mehrere Liganden umfasst; vorzugsweise mindestens einer der Liganden aus CO, Acetylacetonat, Cyclooctadien, Norbornen und Acetat ausgewählt ist.

7. Verfahren nach einem der vorhergehenden Ansprüche, **dadurch gekennzeichnet, dass** es unter einem Druck einer Wasserstoff- und Kohlenmonoxid-Atmosphäre durchgeführt wird, wobei der Druck zwischen 50 bar und 200 bar, vorteilhafterweise zwischen 65 bar und 150 bar, vorzugsweise zwischen 70 bar und 130 bar, speziell zwischen 75 bar und 120 bar, liegt.

8. Verfahren nach einem der vorhergehenden Ansprüche, **dadurch gekennzeichnet, dass** das Molverhältnis zwischen dem Kohlenmonoxid und Diwasserstoff zwischen 2:1 und 1:10 liegt, vorteilhafterweise das Molverhältnis zwischen Kohlenmonoxid und Wasserstoff zwischen 1:1 und 1:5 liegt, vorzugsweise das Molverhältnis zwischen 1:1 und 1:3 liegt, speziell das Molverhältnis zwischen Kohlenmonoxid und Wasserstoff zwischen 1:1 und 1:2 liegt.

9. Verfahren nach einem der vorhergehenden Ansprüche, **dadurch gekennzeichnet, dass** das Molverhältnis zwischen dem Amin und dem Übergangsmetall größer als 20, vorteilhafterweise größer als 50, vorzugsweise größer als 100, speziell größer als 200, ist.

10. Verfahren nach einem der vorhergehenden Ansprüche, **dadurch gekennzeichnet, dass** es sich bei der Zusammensetzung A, die eine oder mehrere Verbindungen der Formel (I) umfasst, um ein pflanzliches Öl mit einer mittleren Zahl von Ungesättigtheiten von weniger als 3,5, vorteilhafterweise weniger als 3,4, vorzugsweise weniger als 3,3, weiter bevorzugt weniger als 3,2, speziell weniger als 3,1, spezieller weniger als 3,0, handelt.

## Claims

1. Process for preparing polyols from a composition A comprising one or more compounds of formula (I)

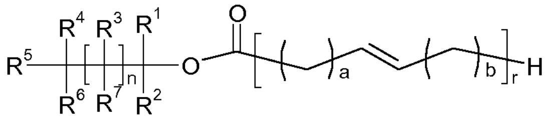

(I)

wherein

$R^1$, $R^2$, $R^3$, $R^4$, $R^5$, $R^6$ and $R^7$ are, independently of one another, and for $R^3$ and $R^7$ independently for each of the n units, chosen from the group consisting of H, $-OR^{15}$, $C_1$-$C_{10}$ alkyl which is unsubstituted or substituted with one or more $-OR^{15}$ groups, $C_6$-$C_{12}$ aryl which is unsubstituted or substituted with one or more $-OR^{15}$ groups, or $C_3$-$C_{10}$ cycloalkyl which is unsubstituted or substituted with one or more $-OR^{15}$ groups, or a group of formula (Ia)

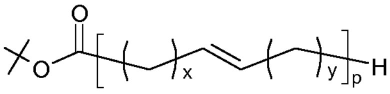

(Ia)

$R^{15}$ represents H or $C_1$-$C_{10}$ alkyl which is unsubstituted or substituted with one or more -OH groups;
a, b, x and y are independently of one another, independently for each group of formula (Ia), independently for each $[(CH_2)_a\text{-C=C-}(CH_2)_b]_r$ unit and independently for each $[(CH_2)_x\text{-C=C-}(CH_2)_y]_p$ unit, an integer between 0 and 20, advantageously between 0 and 15, preferably between 0 and 12;
r is an integer between 1 and 10, advantageously between 1 and 5;
p is an integer between 1 and 10, advantageously between 1 and 5;
n is an integer between 1 and 7;
said process comprising a step a) of placing together, with stirring and under an atmosphere of hydrogen and of carbon monoxide:

- at least one precatalyst which is a complex comprising a transition metal chosen from column 9 of the periodic table,
- a tertiary amine, or a non-quaternary ammonium salt thereof, of formula $NR^8R^9R^{10}$ wherein $R^8$, $R^9$ and $R^{10}$ represent, independently of one another, a $C_1$-$C_{10}$ alkyl, a $C_6$-$C_{12}$ aryl or a $C_3$-$C_{10}$ cycloalkyl, or $R^8$ and $R^9$ form, together with the nitrogen atom to which they are attached, a heterocycle comprising four, five or six ring members,
- said composition A comprising said compound of formula (I).

**2.** Process according to any one of the preceding claims, **characterized in that** step a) is carried out at a temperature between 70°C and 180°C, preferably between 80°C and 150°C.

**3.** Process according to either one of the preceding claims, **characterized in that** said at least one compound of formula (I) is of formula (II)

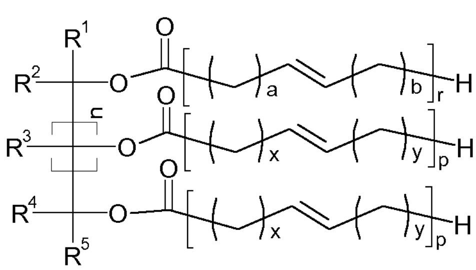

(II)

wherein

a, b, x and y are, independently of one another, independently for each group of formula (Ia), independently for each $[(CH_2)_a\text{-C=C-}(CH_2)_b]_r$ unit and independently for each $[(CH_2)_x\text{-C=C-}(CH_2)_y]_p$ unit, an integer between 0

and 20, advantageously between 0 and 15, preferably between 0 and 12;
p is, independently for each $[(CH_2)_x\text{-}C{=}C\text{-}(CH_2)_y]_p$ unit, an integer between 1 and 5;
r is, independently for each $[(CH_2)_a\text{-}C{=}C\text{-}(CH_2)_b]_r$ unit, an integer between 1 and 5;
$R^1$, $R^2$, $R^3$, $R^4$ and $R^5$ are, independently of one another, and for $R^3$ independently for each of the n units, chosen from the group consisting of H, $-OR^{15}$, $C_1$-$C_{10}$ alkyl which is unsubstituted or substituted with one or more $-OR^{15}$ groups, or a group of formula (Ia) as described above,
$R^{15}$ represents H or $C_1$-$C_{10}$ alkyl which is unsubstituted or substituted with one or more -OH groups;
n is an integer between 1 and 4.

**4.** Process according to the preceding claim, **characterized in that** said at least one compound of formula (I) is of formula (II) wherein

a and x can be, independently of one another, independently for each $[(CH_2)_x\text{-}C{=}C\text{-}(CH_2)_y]_p$ unit and independently for each $[(CH_2)_a\text{-}C{=}C\text{-}(CH_2)_b]_r$ unit, an integer between 1 and 12, advantageously between 2 and 10, preferably between 3 and 9, in particular between 3 and 8 and more particularly between 4 and 8;
b and y can be, independently of one another, independently for each $[(CH_2)_x\text{-}C{=}C\text{-}(CH_2)_y]_p$ unit and independently for each $[(CH_2)_a\text{-}C{=}C\text{-}(CH_2)_b]_r$ unit, an integer between 0 and 12, advantageously between 0 and 10, preferably between 0 and 9, in particular between 2 and 9, and more particularly between 3 and 8;
$R^1$, $R^2$, $R^3$, $R^4$ and $R^5$ are a hydrogen;
p is 1, 2 or 3 independently for each $[(CH_2)_x\text{-}C{=}C\text{-}(CH_2)_y]_p$ unit;
r is 1, 2 or 3 independently for each $[(CH_2)_a\text{-}C{=}C\text{-}(CH_2)_b]_r$ unit;
n is 1.

**5.** Process according to any one of the preceding claims, **characterized in that** the tertiary amine is of formula $NR^8R^9R^{10}$ wherein $R^8$, $R^9$ and $R^{10}$ represent, independently of one another, a $C_1$-$C_{10}$ alkyl, or $R^8$ and $R^9$ form, together with the nitrogen atom to which they are attached, an azetidine, diazetidine, pyrrolidine, imidazolidine or pyrazolidine heterocycle; preferably $R^8$ and $R^9$ form, together with the nitrogen atom to which they are attached, a pyrrolidine.

**6.** Process according to any one of the preceding claims, **characterized in that** the precatalyst is a complex comprising rhodium as transition metal and one or more ligands; preferably, at least one of said one or more ligands is chosen from CO, acetylacetonate, cyclooctadiene, norbornene and acetate.

**7.** Process according to any one of the preceding claims, **characterized in that** it is carried out under pressure of a hydrogen and carbon monoxide atmosphere, said pressure being between 50 bar and 200 bar, advantageously between 65 bar and 150 bar, preferably between 70 bar and 130 bar, in particular between 75 bar and 120 bar.

**8.** Process according to any one of the preceding claims, **characterized in that** the molar ratio between the carbon monoxide and the hydrogen is between 2:1 and 1:10, advantageously the molar ratio between the carbon monoxide and the hydrogen is between 1:1 and 1:5, preferably the molar ratio is between 1:1 and 1:3, in particular the molar ratio between the carbon monoxide and the hydrogen is between 1:1 and 1:2.

**9.** Process according to any one of the preceding claims, **characterized in that** the molar ratio between the amine and the transition metal is greater than 20, advantageously greater than 50, preferably greater than 100, in particular greater than 200.

**10.** Process according to any one of the preceding claims, **characterized in that** said composition A comprising one or more compounds of formula (I) is a vegetable oil having an average unsaturation number of less than 3.5, advantageously less than 3.4, preferably less than 3.3, more preferentially less than 3.2, in particular less than 3.1, more particularly less than 3.0.

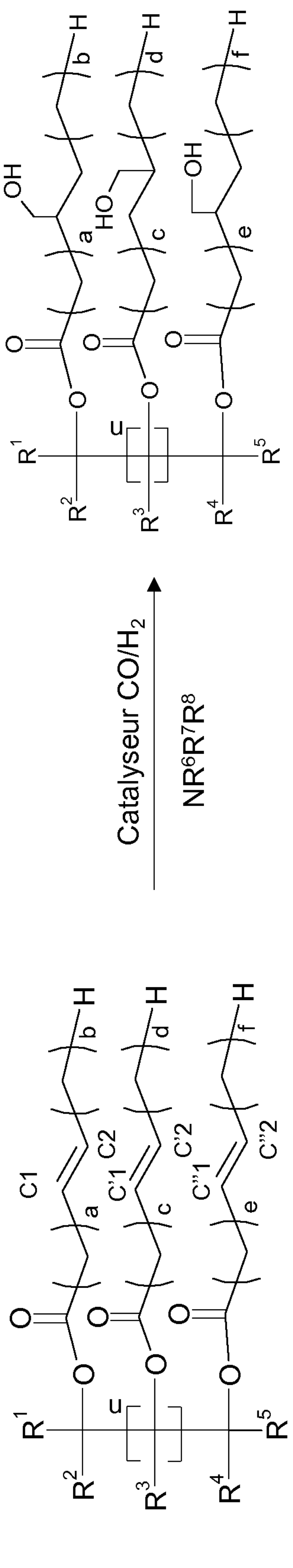


(I)

(II)

Fig. 1

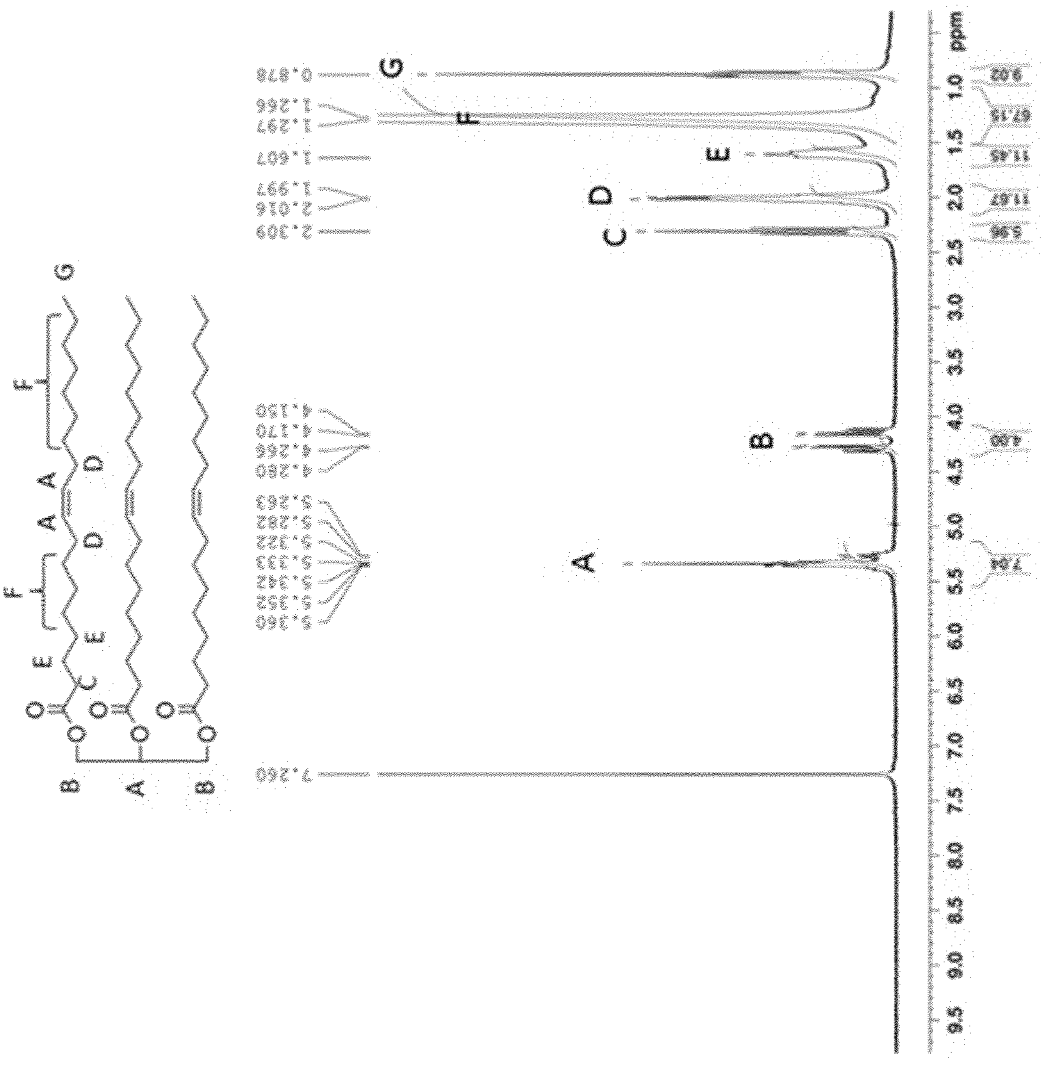
0.878
1.266
1.297
1.607
1.997
2.016
2.309
4.150
4.170
4.266
4.280
5.263
5.282
5.322
5.333
5.342
5.352
5.360
7.260
A
B
C
D
E
F
G
ppm
9.02
67.15
11.45
11.67
5.96
4.00
7.04

Fig. 2

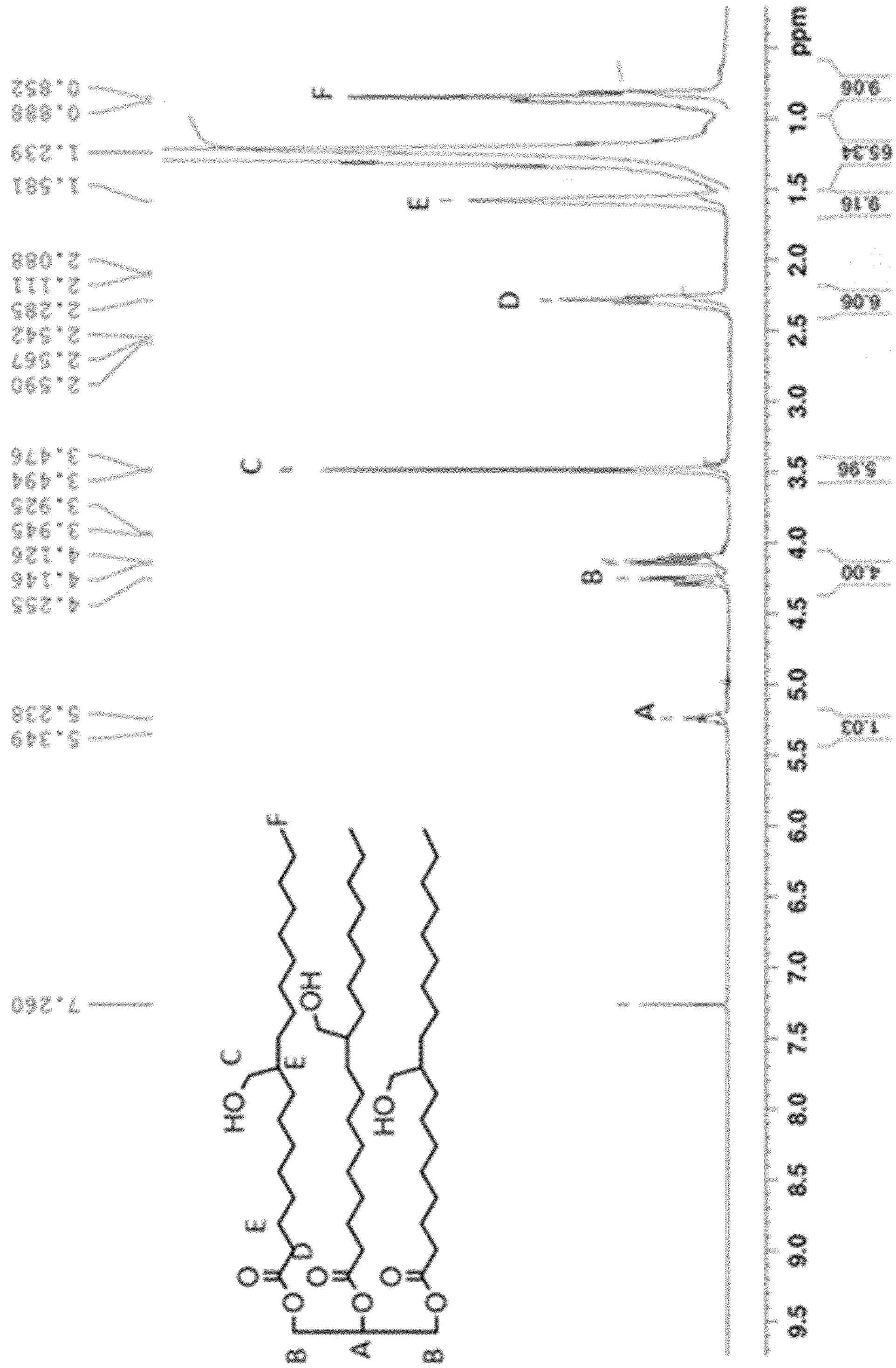

0.852
0.888
1.239
1.581
2.088
2.111
2.285
2.542
2.567
2.590
3.476
3.494
3.925
3.945
4.126
4.146
4.255
5.238
5.349
7.260
9.06
65.34
9.16
6.06
5.96
4.00
1.03
ppm


Fig. 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0014225 A
- US 4197414 A
- WO 20060112344 A
- WO 03093215 A


### Littérature non-brevet citée dans la description


- **MORALES TORRES et al.** *Catal. Sci. Technol.,* 2015, vol. 5, 34-54
- **HUNTER et al.** *Appl. Catal.,* 1985, vol. 19, 275-285
- **ALPER et al.** *Adv. Synth. Catal.,* 2012, vol. 354, 2019-2022